# EUROPÄISCHE PATENTANMELDUNG

(11) **EP 1 262 901 A2**
(43) Veröffentlichungstag der Anmeldung: **04.12.2002**
(21) Anmeldenummer: 02011193.6
(22) Anmeldetag: 21.05.2002
(51) Int. Cl.: G06F 19/00

(54) **Vorrichtung zur automatisierten Risikomodifikation in Risikogruppen**

(30) Priorität: 31.05.2001 DE 10126715
(71) Anmelder: SIEMENS AKTIENGESELLSCHAFT, 80333 München (DE)
(72) Erfinder: Mankopf, Michael, 91096 Moehrendorf (DE); Schmidt, Volker, Dr., 91054 Erlangen (DE)

(57) **Zusammenfassung**

System zur automatisierten Risikomodifikation in Risikogruppen, wobei Erfassungs- und Übermittlungseinrichtungen bei den einzelnen Mitgliedern der Risikogruppe (Patient) zur Ermittlung der relevanten Risikofaktoren und deren jeweiliger Messwerte zur regelmäßigen Weiterleitung an ein Expertensystem, das - in Verbindung mit Datenbanken über mögliche Maßnahmen zur Risikomodifikation - unter Auswertung dieser personenbezogenen Daten Verhaltensempfehlungen und/oder Produktangebot an den Patienten gibt.

## Beschreibung

Die Erfindung bezieht sich auf ein Vorrichtung zur automatisierten Risikomodifikation in Risikogruppen, mit Erfassungsund Übermittlungseinrichtungen bei den einzelnen Mitgliedern der Risikogruppe (Patient) zur Ermittlung der relevanten Risikofaktoren und deren jeweiliger Messwerte zur regelmäßigen Weiterleitung von Risikofaktoren an ein Expertensystem, das - in Verbindung mit Datenbanken über mögliche Maßnahmen zur Risikomodifikation - unter Auswertung dieser personenbezogenen Daten Verhaltensempfehlungen an den Patienten gibt.

In der Bevölkerung existieren Risikofaktoren wie Übergewicht, Fehlernährung, Bewegungsmangel, Nikotin- und Alkoholmissbrauch, Bluthochdruck od. dgl., die über die Zeit zu chronischen Krankheitsbildern führen wie beispielsweise Diabetes, COLD, Herzinsuffizienz, arterielle Verschlusskrankheit, Schlaganfall, Rückenbeschwerden, Gelenkbeschwerden, Lungenkrebs etc.. Durch eine Modifikation der Risikofaktoren kann das Auftreten der genannten Krankheitsbilder verhindert oder zeitlich hinausgezögert werden. Diese Modifikation verlangt jedoch Änderungen des Lebensstils bei Leuten, die typischerweise noch keine Krankheitsanzeichen verspüren und daher nur schwierig zu beeinflussen sind.

Der Schlüssel zur Risikomodifikation beim einzelnen Menschen ist individuell unterschiedlich. Dementsprechend wurden bisher auch von verschiedenen Seiten mit unterschiedlichen Ansätzen Regelfälle derartiger Risikomodifikationen gesucht wie beispielsweise Aufklärungskampagnen in den öffentlichen Medien oder Patienteninformation über Apotheken, beim Arztbesuch oder aus dem Internet. Darüber hinaus existieren vielfältige andere Gesundheitsprogramme, wie beispielsweise die weight watcher, die speziell die Risikofaktoren wie Übergewicht und Fehlernährung bekämpfen.

In der US 5,967,789 A ist bereits eine Vorrichtung der eingangs beschriebenen Art vorgeschlagen worden, bei der der Patient mit einem Expertensystem kommuniziert, das ihm aufgrund seiner speziellen Risikofaktoren Verhaltensempfehlungen gibt. Diese Verhaltensempfehlungen umfassen aber keine speziellen Produktangebote, was der Patient essen und trinken sollte, und in welcher Art und Weise er sich möglicherweise sportlich wie betätigen kann und insbesondere wird hierbei auch überhaupt keine Beurteilung dahingehend gemacht, wie teuer der jeweilige Verhaltensvorschlag kommt, und welche Kosten dadurch auf die an einem solchen erfindungsgemäßen Risikomodifikationssystem interessierten Krankenkassen zukommt.

Eine aus der US 5,486,999 A bekannt gewordenen Vorrichtung zur Auswahl von Behandlungsstrategien und Verhaltensempfehlungen für Patienten, wobei es darum geht, für dessen spezielle Problematik ungeeignete Vorschläge und Behandlungen zu vermeiden, soll zwar zur Kostensenkung beitragen, dies aber speziell nur dadurch, dass ungeeignete Empfehlungen vermieden werden.

Der Erfindung liegt die Aufgabe zugrunde, eine Vorrichtung der eingangs genannten Art so auszugestalten, dass das Expertensystem aus einer Vielzahl unterschiedlich möglicher Verhaltensempfehlungen und Produkten, die dem Risikopatienten in seiner Situation weiterhelfen könnten, eine zweckentsprechende Auswahl trifft.

Zur Lösung dieser Aufgabe ist erfindungsgemäß vorgesehen, dass das Expertensystem, in dessen Datenbanken eine Vielzahl von Produktangeboten gespeichert sind, eine Bewertungseinrichtung umfasst, die unterschiedliche mögliche Verhaltensempfehlungen nach den entstehenden Kosten beurteilen und optimieren kann und dass das Expertensystem derart eingerichtet ist, dass es zunächst einfache und billige Verhaltensempfehlungen oder Produktangebote gibt und erst bei aus Folgemeldungen erkannter Erfolglosigkeit früherer Vor-Empfehlungen stufenweise aufwändigere und/oder teurere Verhaltensempfehlungen oder Produktangebote vorschlägt.

Durch die erfindungsgemäße Ausgestaltung wird der Patient nicht mit einer Vielzahl von Ratschlägen und Produktempfehlungen versorgt, aus denen er gegebenenfalls auch erst noch selbst eine Auswahl treffen muss, sondern es wird ihm jeweils ein Verhalten empfohlen und ein ganz spezielles Produkt angeboten, die geeignet wären, seine Beschwerden zu lindern bzw. sein Risiko herabzusetzen und war auf der Basis, dass zunächst mit sehr einfachen und billigen Empfehlungen gearbeitet wird und teuere Behandlungsmethoden und Behandlungsprodukte nur dann stufenweise angeboten und eingesetzt werden, wenn die billigeren Varianten für den angestrebten Zweck der Risikoverminderung nicht ausreichend waren.

Der Patient - der lediglich abgekürzt so bezeichnet wird, obgleich im eigentlichen Sinn noch keine behandlungsbedürftige Erkrankung vorliegt - oder Angehörige, Eltern oder Pfleger, erfassen die jeweiligen Risikofaktoren, zum Beispiel den Blutdruck, das Körpergewicht, den Zigarettenkonsum, den Alkoholkonsum, Beschwerden des Bewegungsapparates oder körperliche Leistungsfähigkeit usw. mit entsprechenden Messgeräten. Diese können sein Messgeräte im eigentlichen Sinn wie beispielsweise Blutdruckmessgeräte, Waagen od. dgl., oder aber auch Fragebogen, mit Hilfe denen nicht nur etwaige Risikofaktoren abgefragt werden sondern mit denen auch spezielle Wunschvorstellungen, Projektziele, bevorzugte Nahrungsmittel od. dgl. abgefragt werden können, die über die Funktion als Risikofaktor hinaus auch wesentliche Informationen für das Expertensystem dahingehend liefern, dass es individuell angepasste gewissen Wunschvorstellungen des Patienten nahekommende Empfehlungen erarbeiten kann. Derartige den Neigungen des Patienten entgegenkommende Empfehlungen, also beispielsweise Ernährungsvorschläge, die nicht gerade spezielle Fischgerichte beinhalten, wenn man durch die Befragung des Patienten weiß, dass er partout niemals Fisch ist, haben erheblich größere Chancen vom Patienten akzeptiert zu werden, so dass demzufolge auch die Erfolgschancen der automatisierten Risikomodifikation steigen.

Die erfassten Messwerte werden vom Patienten an das zentrale System über die vorhandenen Übermittlungsvorrichtungen gesandt, zum Beispiel email, Fax, Telefon mit Spracherkennung, DTMF, Internet od. dgl. und vom zentralen Expertensystem zunächst in eine verarbeitbare Form gebracht. Gegebenenfalls kommen hier Spracherkennung, OCR Software zum Einsatz, um aus den eingesandten Informationen automatisch elektronisch verarbeitbare Daten zu erzeugen.

Die Daten können in einer "Gesundheitsakte" gespeichert werden. Das zentrale Expertensystem verarbeitet die Informationen und erzeugt einen Vorschlag zur Risikomodifikation, den es an den Patienten übermittelt, wozu der Vorschlag mit Verhaltensempfehlungen und/oder Produktangeboten natürlich in ein für den Patienten lesbares Format gebracht werden muss. Als Kommunikationskanäle zum Betroffenen stehen dabei die gleichen Kanäle zur Verfügung wie für die Übermittlung der Daten vom Patienten zum Expertensystem, also wiederum email, Fax, Text2Speech, Webpages, XML, HTML. Bevorzugt generiert das Expertensystem die Vorschläge jeweils im gleichen Format und sendet es auf dem gleichen Weg, auf dem es die Risikofaktoren und Messwerte vom Patienten erhalten hatte.

Wenn der Vorschlag vom Patienten angenommen wird und zu dem gewünschten Erfolg führt, so erkennt dies das System durch Überwachung der vom Patienten ja regelmäßig immer wieder übermittelten Gesundheitsparameter.

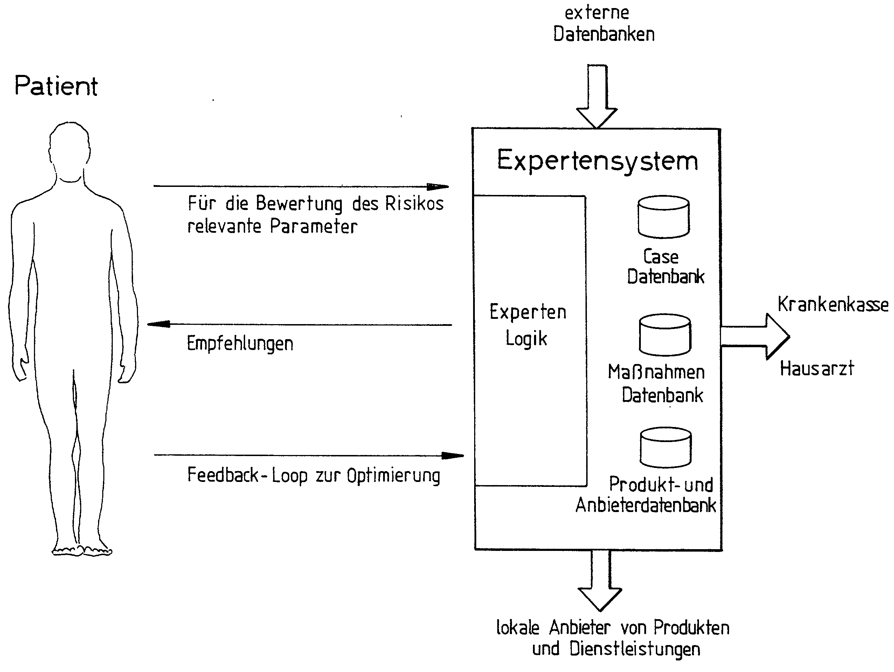

Ändern sich diese Parameter jedoch nicht in der gewünschten Weise, so generiert das System einen alternativen Vorschlag und sendet ihn an den Betroffenen (Feedback loop). Ein Ablaufdiagramm des erfindungsgemäßen Systems ist in der Zeichnung schematisch dargestellt.

Die Generierung eines Vorschlages erfolgt durch ein Expertensystem, das insbesondere auch Vorschläge zur Modifikation mehrerer Risiken erzeugen kann. Dieses Expertensystem kann ein System sein, das mit festen Regeln arbeitet oder ein lernendes System, das zunächst von statistischen Daten ausgeht und lernt, welche Maßnahmenkombinationen beim betroffen Individuum zum Erfolg führen. Dazu greift es auf unterschiedliche Datenbanken zu, die generische Maßnahmen enthalten, wobei anschließend die Rohvorschläge zum einen an das Individuum angepasst und zum anderen nach Kosten optimiert werden können. Dazu werden individuelle Gesundheitsdaten verwendet, zum Beispiel Daten zu persönlichen Vorlieben, körperliche Merkmale, gemessene Gesundheitsparameter usw..

Aus der Datenbank mit lokalen Anbietern und deren Leistungen erzeugt das Expertensystem dann den endgültigen Vorschlag, der an den Betroffenen über unterschiedliche Medien übersandt werden kann, wobei das Expertensystem gegebenenfalls auch auf bereits existierende Expertensystemmodule, auch über Internet, zugreifen kann, die bereits isoliert existieren, wie zum Beispiel Expertensystemmodule über Diätetik.

Eine Verhaltensempfehlung des Expertensystems kann im einfachsten Fall eine spezifische Information für den Betroffen (ähnlich der Aufklärungsinformation - zum Beispiel: "Sie sind zu schwer, ihr persönliches Idealgewicht ist ...") sein, aber auch ein Plan, um über die Zeit einen Gesundheitsparameter in den angestrebten Zielbereich zu bringen. Ein Beispiel ist die Normalisierung des Blutdrucks und des Körpergewichtes in einem Projektplan mit einer sich ständig verfeinernden Folge von Verhaltensempfehlungen:
1. Vorschlag:
   Aufklärung des Betroffenen über sein individuelles Risiko.
2. Vorschlag:
   Essensplan zur Gewichtsreduktion und Kochsalzrestriktion.
3. Vorschlag:
   Vorschlag 2 plus individuell angepasste Kochrezepte.
4. Vorschlag:
   Vorschlag 3 mit zusätzlicher Bestellung über das Expertensystem im Supermarkt zur Lieferung der entsprechenden geeigneten Zutaten.
5. Vorschlag:
   Wie Vorschlag 4 mit Einschaltung eines Lieferservices für fertig zubereitetes Essen.
6. Vorschlag:
   Vorschlag 5 sowie ein zusätzliches Bewegungsprogramm.
7. Vorschlag:
   Vorschlag 6 sowie Einschaltung lokaler Anbieter von Fitnessprogrammen, von denen er beispielsweise ein Gutschein für ein Probetraining und ein für ihn speziell angepasstes Fitnessprogramm erhält.

Speziell bei der Einschaltung lokaler Leistungserbringer zur Lieferung von Nahrungsmitteln oder über Fitnessprogramme kann ein Teil der Kosten des erfindungsgemäßen Systems auch aus direkt mit dem System zu verrechnenden Provisionen dieser lokalen Anbieter erbracht werden. In erster Linie sind an einem solchen System aber Krankenkassen und Gesundheitsbehörden interessiert, die durch eine erfolgreiche automatisierte Risikomodifikation in Risikogruppen ganz erhebliche Kosten bei der Behandlung der möglicherweise aus diesen Risikofaktoren resultierenden Krankheiten einsparen können. Aus diesem Grund kann das Expertensystem - in Absprache selbstverständlich mit dem Patienten - eine Informationsweiterleitung an andere Stellen wie Krankenkassen, Hausarzt od. dgl. veranlassen.

## Patentansprüche

1. Vorrichtung zur automatisierten Risikomodifikation in Risikogruppen, mit Erfassungs- und Übermittlungseinrichtungen bei den einzelnen Mitgliedern der Risikogruppe (Patient) zur Ermittlung der relevanten Risikofaktoren und deren jeweiliger Messwerte zur regelmäßigen Weiterleitung von Risikofaktoren an ein Expertensystem, das - in Verbindung mit Datenbanken über mögliche Maßnahmen zur Risikomodifikation - unter Auswertung dieser personenbezogenen Daten Verhaltensempfehlungen an den Patienten gibt, **dadurch gekennzeichnet, dass** das Expertensystem, in dessen Datenbanken eine Vielzahl von Produktangeboten gespeichert sind, eine Bewertungseinrichtung umfasst, die unterschiedliche mögliche Verhaltensempfehlungen nach den entstehenden Kosten beurteilen und optimieren kann und dass das Expertensystem derart eingerichtet ist, dass es zunächst einfache und billige Verhaltensempfehlungen oder Produktangebote gibt und erst bei aus Folgemeldungen erkannter Erfolglosigkeit früherer Vor-Empfehlungen stufenweise aufwändigere und/oder teurere Verhaltensempfehlungen oder Produktangebote vorschlägt.

2. System nach Anspruch 1, **dadurch gekennzeichnet, dass** über die Erfassungseinrichtungen auch persönliche Wunschvorstellungen, Projektziele, Essensgewohnheiten etc. mit erfasst und an das Expertensystem weitergeleitet werden.

3. System nach Anspruch 1 oder 2, **dadurch gekennzeichnet, dass** die Übermittlungsvorrichtungen zum Beispiel email, Fax, Telefon mit Spracherkennung, DTMF, Internet od. dgl. umfassen.
